# EUROPEAN PATENT APPLICATION

(11) **EP 4 278 995 A1**
(43) Date of publication of application: **22.11.2023**
(21) Application number: 22173887.5
(22) Date of filing: 17.05.2022
(51) Int. Cl.: A61B 17/3203, A61B 18/04

(54) **TREATMENT DEVICE**

(71) Applicant: CARAG AG, 6340 Baar (CH)
(72) Inventor: CHRISTEN, Lukas, 6004 Luzern (CH); GOOD, Roman, 8048 Zürich (CH); LARSSON, Michael, 6302 Zug (CH); KESSLER, Jonas, 6005 Luzern (CH); BERNHARD, Jérôme, 8003 Zürich (CH)
(74) Representative: Grünecker Patent- und Rechtsanwälte PartG mbB

(57) **Abstract**

The present invention relates to an improved treatment device e.g. for treating wounds and other tissue with a hand piece (112) comprising a nozzle (18), which nozzle (18) is in fluid communication with a pump (100), which pump (100) is in fluid communication with a reservoir (104) of fluid to be jetted onto the treatment area. For enhancing treatment success, the device of the invention has heating means (16, 30), which are adopted to heat the fluid.

## Description

The present invention relates to a treatment device, in particular a device for treating wounds or other tissue. The device has a hand piece comprising a nozzle, which nozzle is in fluid communication with a pump, which pump is in fluid communication with a reservoir of fluid to be jetted onto the wound.

Such device is generally known e.g. from the WO 2015/032863 A1, EP 3 097 876 A1 or US 2018/0126392 A1.

The device known from prior art is used as a surgical tool for debridement by use of a fluid jet. In such therapy, a microfluid jet is sprayed onto the wound surface of a wound of a life being, usually a human being, in order to remove tissue or secretion. The fluid jet is generally a treatment fluid which is formed, for example, predominantly or exclusively from water or a physical saline solution. The fluid jet must fulfill special requirements in order to be effective in the wound. It must have a small beam diameter and extend predominantly in a coherent manner over the relatively long distance in order to impact as targeted or focused as possible onto the wound with a predetermined pressure sufficient to remove tissue from the wound surface.

This requirement also applies to the inventive device.

The present invention aims to provide an improved treatment device.

As a solution to this object, the present invention proposes to provide the device with at least one heating means adapted to heat the fluid.

The device of the present invention may be used for e.g. a surgical treatment by use of a heated fluid jet e.g. for debridement, tissue dissection, cauterization and/or disinfection.

The heating means are preferably adapted to heat the fluid to a temperature of above 80°C at the downstream side of the nozzle, i.e. at the exit of the fluid to be impinged on the wound. This temperature is the temperature of the entire fluid jet stream. The present device allows to apply heated fluid including steam or vapor on the surface of the wound or tissue. Such heated fluid may disinfect the wound. Further, local overheating of the surface of the wound may be beneficial for the further growth of tissue within the wound which is required for finally healing the wound.

The heated fluid may continuously or intermittently be applied to the wound. In case of an intermittent application, heated fluid will be released from the device and jetted onto the wound intermittent with the focused fluid jet described above for the debridement. Such debridement may lead to impingement of the wound with a fairly high pressure with a highly focused fluid jet. The fluid jet results from the reservoir, which reservoir is usually used as a source for the fluid to be heated. In the therapy the heated fluid may be jetted onto the wound and/or be applied to the wound in form of steam or vapor having a rather low impact on the wound.

The pressure applied within the apparatus to advance the fluid towards the nozzle may be 40 bar or more. For a nozzle with a diameter of 0.08 mm, the pressure within the apparatus should be between 60 and 120 bar; for a nozzle with a diameter of 0.10 mm, the pressure within the apparatus should be between 40 and 120 bar and for a nozzle with a diameter of 0.125 mm, a pressure of between 20 and 120 bar may be applied to the fluid within the apparatus. Those conditions may apply to debridement, only, i.e. a jet of unheated water impinged on the tissue for debridement by the water jet. Those conditions may likewise apply to the treatment with heated fluid.

For a treatment utilizing a heated fluid, the applied pressure may be less. Such treatment may in particular be applied for the denaturation of proteins at least on the surface of the tissue. The amount of denaturation and the depth of the denatured tissue greatly depends on the treatment time and the distance of the nozzle from the surface to be treated. It also depends on the power applied to the heating means. In case the power applied to the heating means, which power usually is an electrical power, is too low, droplets of heated water will be impinged on the surface to be treated, which is usually not as advantageous as the application of a rather dry steam, i.e. steam without any droplet. Experiments carried out by the inventors showed, that the ratio between the electrical power uptake of electrical heating means and the energy taken up by the fluid should preferably exceed 180, more preferably should exceed 220, most preferably should exceed 250, even more preferred should exceed 300.

The energy taken up by the fluid is the product of the mass flow, the specific heat capacity of the fluid and the difference between the fluid temperature downstream of the heating means and upstream of the heating means

The heating means should be adapted to provide steam having a temperature of at least 75°C, preferably 85°C, more preferably 95°C, each temperature being measured 10 mm downstream from the nozzle. 10 mm +/- 2 mm may as well be a suitable range for the distance between the nozzle and the tissue to be treated when using the inventive device in therapy.

The flow rate of fluid to be heated, in particular water, within the divide may the 25 ml/min or more. For a flow rate of 25 ml/min an electric power of at least 450 W shall be applied. For a flow rate of 50 ml/min an electric power of at least 700 W shall be applied. For a flow rate of 75 ml/min an electric power of at least 850 W shall be applied. For a flow rate of 100 ml/min an electric power of at least 1100 W shall be applied. At present, a flow rate of between 50 to 75 ml/min seems to be the most promising range for therapeutic applications. For properly heating a mass flow of 10 g/min the electric power of about 200 W should be applied., Which electric power should be arrayed by 100 w +/- 10 W for every increase in the a mass flow of 10 g/min.

In the inventive method, the surface of the wound may be heated for 1 seconds to 60 seconds with a temperature on the surface of the wound of between 45°C and 100°C. Such treatment may last between 1 seconds and 60 minutes. The higher the flow rate with a sufficiently dry steam, the lower the duration of the treatment can be.

The treatment preferably is to provide heat conditions on the skin or in the wound above the point of denaturation of proteins.

Both, the heated fluid and the fluid jet are released preferably through an identical nozzle geometry, which nozzle may have a length usually of not more than 30 mm and an internal cross section providing the flow passage for the fluid of between 0.15 and 0.6 mm, preferably between 0.25 and 0.35 mm. Such nozzle is usually a metallic nozzle having a length piece of a small diameter tube and a nozzle element usually welded to such piece of tube as described in US 2018/0126392 A1. Regarding the details of the device for impinging a fluid jet on the wound ground for debridement, disclosure of this prior art is incorporated herein by reference. The aperture of the nozzle element releasing the fluid jet into atmosphere has a diameter or size of not more than 0.2 mm, preferably of between 0.03 mm and 0.2 mm. The same applies to EP 2 251 142 A1, which discloses an alternate device for impinging a fluid jet on the wound ground. The details of the device disclosed in this prior art may - in addition to heating means - be provided in an embodiment of the present invention.

In order to effectively heat the fluid just upstream of the opening of the nozzle, the heating means are preferably received within the hand piece. On a regular basis, the heating means terminate just upstream of the nozzle. Effective heating usually requires a heating capacity of at least 200 WATT, preferably at least 1000 WATT. Thus, the heating means are to extend a considerable length along the fluid path within the hand piece, which length is usually at least 50 mm.

The device of the present invention may have at least one or plural heating means. These heating means are usually provided upstream of the nozzle and preferably received within the hand piece.

Alternatively, or additionally, a heating means may be provided upstream of the hand piece, specifically provided between the hand piece and the pump. Such heating means may for example be flexible to surround and/or be received in a tube, in particular a flexible hose connecting the pump with the hand piece.

The heating means may be provided inside the pump or assigned to the tubes, specifically can be provided surrounding the pump housing.

Moreover, heating means may be provided upstream of the pump. In particular, heating means may be provided between the pump and the reservoir. Specifically, the heating means may be assigned to the reservoir to preheat the fluid of the reservoir prior to advancing the fluid towards the nozzle.

The inventive device may have more than one heating means. Heating means may be provided at each of the aforementioned locations and/or regions. Any heating means may contribute to a temperature rise of the fluid and/or avoid thermal loss. Moreover, a thermic insolation may be provided surrounding one or more heating means upstream of the nozzle. Such thermic isolation protects the user of the device during use and prevents heat loss of the fluid along the fluid path from the first heating means in flow direction to the nozzle. "Upstream" relates to the flow of fluid within the device towards the nozzle.

For improved heat transfer, the heating means preferably surround the path of fluid within the hand piece. Accordingly, the heating means extends circumferentially around this path and extends in longitudinal direction of this path. Alternatively, the at least one heating means is surrounded by a fluid path of the fluid. Thus, the fluid can be in direct contact with the heating means and will likewise provide a thermal insulation between the heating means and the outer circumference of the device or a portion thereof, which will improve efficiency and simplify handling.

The heating means may be formed by a resistance heater and/or an induction heater, an infrared heater, a plasma heater, a microwave heater, a thin-film or a thick-film heater. For heating by induction, an induction coil may be provided around a core, which induction coil creates an electric magnetic field to induce heat into the core. In case of a resistance heater, a heating wire may be provided around the core. Alternatively, the core may be surrounded or formed by a PTC-material. Both, the PTC-material or the heating wire are connected to different electrical polarities to generate heat via resistance of the material. With a small lumen within the core as a path of the fluid, such path may be drilled through a PTC-material in order to improve dissipation of the generated heat from the PTC-material into the fluid to be heated.

In case of a heating wire, the same is usually wound around the core, which core surrounds the fluid path of the fluid within the hand piece. The core usually has a bore for guiding the fluid through the core. This bore may receive a tube providing the path of the fluid. Preferably and in order to reduce the path of the heat traveling from the heat source to the fluid, the bore forms the fluid path, i.e. the walls of the bore are in use wetted by the fluid in use. On the outer circumference of the bore, a helical groove is provided. Such helical groove may be provided in form of threads or the like. The helical groove will be wound around a considerable longitudinal length of the core. The heating wire mentioned above will be wound around the core in circumferential direction. Moreover, the resistance heating wire is received within the coral groove thereby adjusting the axial distance of adjacent windings of the wire on the outer circumference of the core and reducing the radical distance between the heat-emitting wire and the fluid path, which is usually provided in the centre of the core.

For electrically isolating the various windings of the heat resistant wire, the core preferably is coated with an electrically insulating material. Additionally or alternatively, the heating wire may be coated with an electrically insulating material. For the same purpose, the core may be made of an electrically insulating material. Making the core of ceramic will reduce the risk of an electric short circuit. A thermal conductive material may be applied between the resistance heating wire and the core. In particular, the thermal conductive material should fill out any voids between the resistance heating wire and the core.

According to a preferred embodiment of the present invention which allows to utilize components of an existing nozzle for spraying a fluid jet onto the wound to effect debridement, the device has a nozzle component and a connector component, which are basically known from prior art US 2018/0126392 A1. In this prior art device, the nozzle component comprises a nozzle element, which nozzle element preferably is secured by injection molding a thermoplastic material around the metallic tube of the nozzle element. The connector component is a separate component provided to be connected to a tube, which fluidically connects the hand piece with the pump. The core already mentioned above is interdisposed between the nozzle component and the connector component. The core is surrounded by the heating means. The nozzle component and the connector component are usually not surrounded by heating means. The nozzle component and the connector component are usually two separate parts which are connected to the core in a fluid tight manner. The core may be welded and/or glued and/or threaded against the nozzle component and/or the connector component. Those elements for technically effecting fluid communication between the pump and the nozzle may be surrounded by an outer sleeve or the like which is adapted to manually hold the device during use. The sleeve enhances the diameter of the hand piece. Furthermore, the sleeve usually surrounds a thermal insulation between at least the heating means within the hand piece and the outer circumferential surface thereof which is manually held in use.

In order to improve thermal performance of the device, the same may have a preheater arranged upstream of the hand piece for pre-heating the fluid. Preferably, such preheater may be assigned to the reservoir in order to lift the temperature of the fluid in the reservoir above room temperature.

The present invention may now be described by referring to different embodiments elucidating the inventive concept in combination with the drawings. In the drawing
- Figure 1: is a three dimensional cross-sectional view of essential components of a first embodiment, where
- Figure 1a: is an enlarged cross-sectional view of a nozzle component;
- Figure 1b: is an enlarged cross-sectional view of a downstream end of the core of the embodiment of Figure 1;
- Figure 2: is a perspective side view of a second embodiment of essential components of the hand piece;
- Figure 3: is a perspective view of an embodiment of a device for treatment of wounds;
- Figure 4: is a three dimensional cross-sectional view of essential components of a first embodiment, where
- Figure 4a: is an enlarged cross-sectional of detail Z in Figure 4;
- Figure 4b: is an enlarged cross-sectional of detail X in Figure 4 and where
- Figure 4c: is an enlarged perspective side view of the downstream end of the heating means.

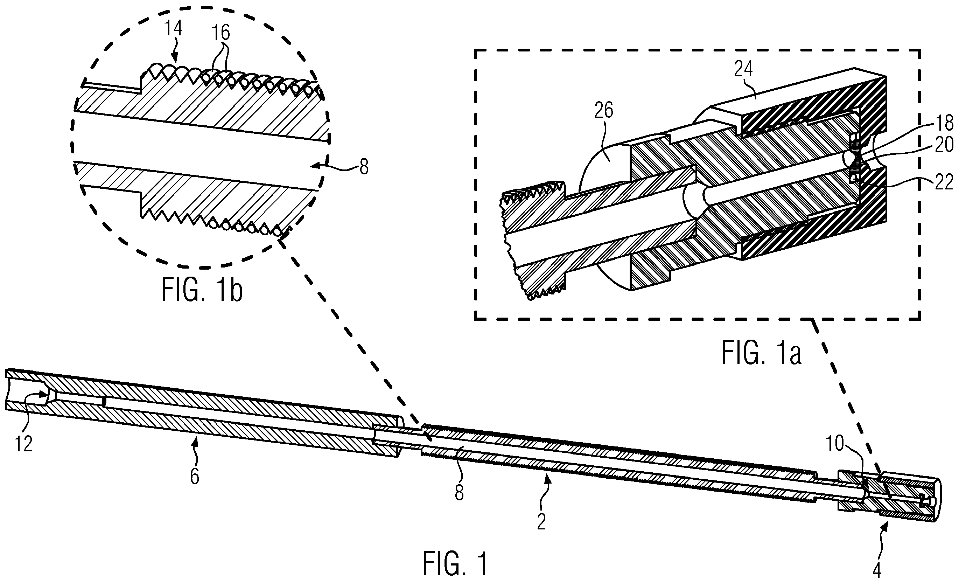

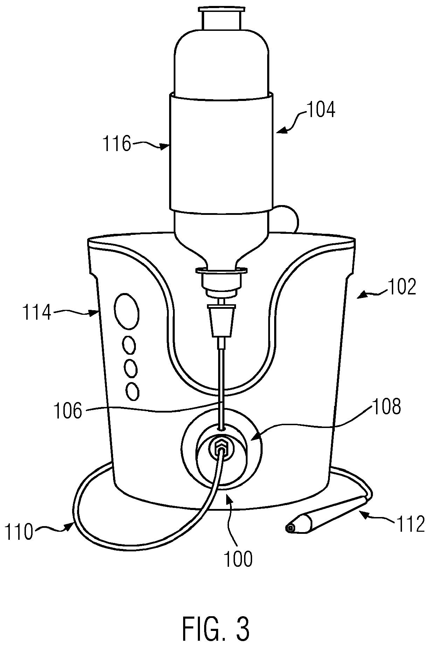

In Figure 1 reference numeral 2 identifies a core interdisposed between a nozzle component 4 and a connector component 6, wherein the nozzle component 4, the connector component 6 and the core 2 are individual elements. The nozzle component 4 may be designed in accordance with the embodiment US 2018/0126392 A1 or EP 2 251 142 A1. The component 6 is provided with a hose received within the connector housing. A downstream end of the core 2 is received within and secured to the nozzle component 4. This core 2 has a central bore 8 guiding the fluid through the core 2. This bore 8 is aligned with a fluid path 10 provided within the nozzle component 4 and a fluid path 12 provided within the connector component 6. The downstream end of this connector component 6 has an enlarged bore for receiving and connecting a tube connecting the embodiment of Figure 1 with a pump (see Figure 3).

Figure 1b elucidates a helical groove provided in circumferential and axial direction of the core 2, which helical groove is identified with reference numeral 14. The helical groove of 14 has plural windings extending in circumferential direction around the core 2. Within the helical groove 14 there is provided a resistance heating wire 16 coated with an electrically insulating material. As derivable from Figure 1b, the bore 8 is wetted by the fluid, i.e. a separate tube or the like for guiding the fluid is missing. This resistance heating wire 16 forms a first embodiment of heating means of the present invention.

Figure 1a shows details of the nozzle component 4, which details. The nozzle component 4 comprises a nozzle 18 formed by an orifice provided within a nozzle saphire 20 surrounded by an O-Ring as described in EP 2 251 142 A1. Downstream of the nozzle 18 a cap section of a nozzle sleeve 24 grasps over the nozzle saphire 20 to retain respective nozzle saphire 20 within the nozzle component 4. This nozzle sleeve 24 is threaded against a nozzle element 26, which nozzle element 26 is connected to the core 2, which core 2 is received in a receptacle of the nozzle element 26. The connection may be secured by welding or soldering.

After joining the core 2, the nozzle component 4 and the connector component 6, electrically and/or thermal insulating material is provided around the circumference at least of the core 2. After that and after joining the tube and the connector component 6, the unit will be encompassed within a housing, which housing will radially surround at least the core 2. Preferably, the housing will fully surround in axial and radial direction the core 2, the nozzle component 4 and the connector component 6. The housing is usually made of two different parts which sandwich in between the core and the components 4, 6. After that, the hand piece is radially manufactured. The hand piece is usually a disposable product.

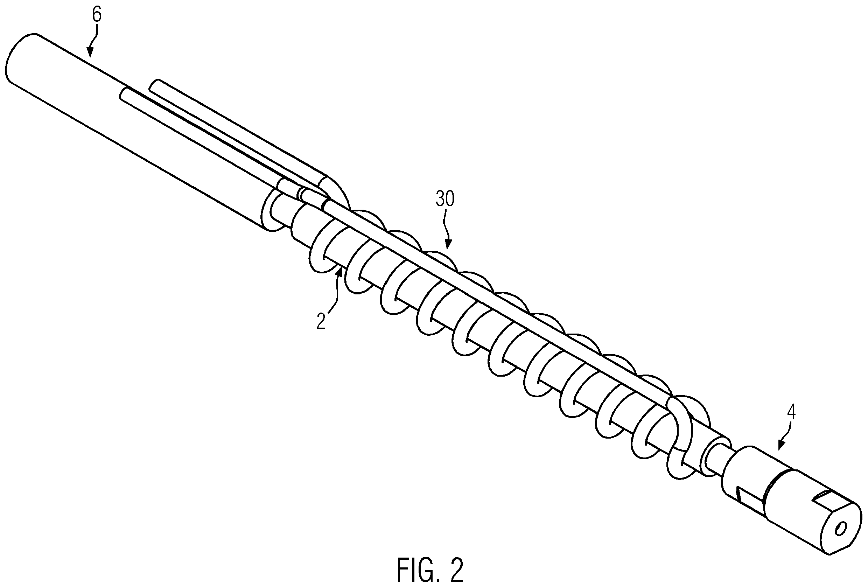

Figure 2 shows an alternate embodiment. Similar parts are provided with similar reference numbers. The major difference in comparison to Figure 1 is the provision of an inductive heater 30 helically provided around the circumference and length of the core 2, which inductive heater 30 forms a second embodiment of heating means of the present invention.

The device is generally shown in Figure 3 and comprises a pump 100 received within a pump housing 102, which supports a reservoir 104. This reservoir 104 through a first tube 106 is in fluid communication with a pump module 108, which pump module 108 is generally described in EP 3 258 111 A1. The downstream end of the pump module 108 is connected to a second tube 110, which is received in and connected with the connector component of a hand piece 112. The pump housing 102 has an operational input section 114 with plural buttons for setting parameters influencing the performance of the device. Within the pump housing 102, a control element is provided for controlling the operation of the pump module 108 as well as the operation of the heating means 16, 30. The user may e.g. select an intermittent application of a fluid jet for debridement and heated fluid and/or vapor for desinfection or affecting local overheating within the wound ground. The pump housing 102 usually has an interface for electrically connecting the hand piece 112 and the heating means 16, 30 contained therein with the control and energy supply for the heating within the pump housing 102.

Figure 3 furthermore identifies a preheater 116, which preheater 116 circumferentially surrounds the reservoir 104 for preheating the fluid contained therein. For control and power supply, said preheater 116 is likewise connected with the pump 100 via an interface (not shown).

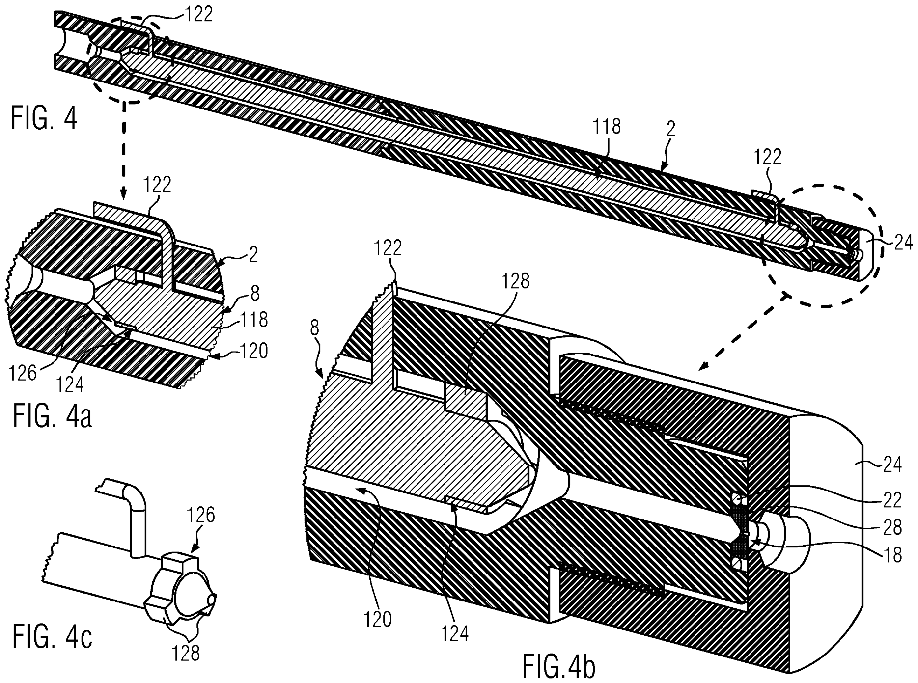

The figures 4, 4a, 4b and 4c show an alternative embodiment. Parts which have been realized and discussed in the previous embodiment are identified with reference numbers as before. In this alternative embodiment a centrally arranged heating core 118 is received within the bore 8 to provide an annular shaped flow channel 120 around the heating core 118. The heating core 118 is energized by two electrical contact elements 122 being assigned to different polarity, extending through the core 2 in radial direction and being bent to extend in axial direction of the core 2 on the outer circumference thereof. An upstream end and a downstream end of the heating core 118 is provided with a circumferential groove 124, each receiving a ring shaped support element 126 having three circumferentially disposed supporting legs 128, which supporting legs 128 contact the inner circumferential surface of the core 2 to concentrically arrange the heating core 118 within the core 2 and to provide axial access to said flow channel 120 at the upstream and the downstream end of the heating core 118 - see in particular Fig 4b.

### List of reference numbers

- 2: core
- 4: nozzle component
- 6: connector component
- 8: bore
- 10: fluid path of the nozzle component 4
- 12: fluid path of the connector component 6
- 14: helical groove
- 16: resistance wire
- 18: nozzle
- 20: nozzle saphire
- 22: O-Ring
- 24: nozzle sleeve
- 26: nozzle element
- 30: inductive heater
- 100: pump
- 102: pump housing
- 104: reservoir
- 106: first tube
- 108: pump module
- 110: second tube
- 112: hand piece
- 114: operational input section
- 116: preheater
- 118: heating core
- 120: annular shaped flow channel
- 122: contact element
- 124: circumferential groove
- 126: support element
- 128: supporting leg

## Claims

1. A treatment device with a hand piece (112) comprising a nozzle (18), which nozzle (18) is in fluid communication with a pump (100), which pump (100) is in fluid communication with a reservoir (104) of fluid to be jetted onto the treatment area **characterized by** at least one heating means (16; 30) adopted to heat the fluid.

2. The device according to claim 1, **characterized in that** the at least one heating means (16; 30) are provided upstream of the nozzle (18).

3. The device according to claim 1 or 2, **characterized in that** the at least on heating means (16; 30) is received within the hand piece (112).

4. The device according to claim 1 or 2, **characterized in that** at least one of the heating means (16; 30) are provided upstream of a hand piece (112).

5. The device according to claim 3 or 4, **characterized in that** at least one of the heating means (16; 30) are provided between the hand piece (112) and the pump (100).

6. The device according to any of the preceding claims, **characterized in that** at least one of the heating means (16; 30) are provided upstream of the pump (100).

7. The device according to claim 6, **characterized in that** the at least one of the heating means (16; 30) are provided between the pump (100) and the reservoir (104).

8. The device according to claim 6 or 7, **characterized in that** the at least one of the heating means (16; 30) are assigned to the reservoir (104).

9. The device according to any of the preceding claims, **characterized in that** the at least one of the heating means (16; 30) surrounds a fluid path of the fluid.

10. The device according to any of the preceding claims, **characterized in that** the at least one of the heating means (16; 30) is surrounded by a fluid path of the fluid.

11. The device according to any of the preceding claims, **characterized by** a thermal insulation surrounding the at least one heating means (16; 30) and/or the hand piece (112) and/or the pump (100) and/or the reservoir (104) and/or any fluid path downstream of the hand piece (112).

12. The device according to any of the preceding claims, **characterized in that** the at least one of the heating means is selected from the group containing a resistance heater (16), an infrared heater, a plasma heater, a microwave heater, a thin-film heater, a thick-film heater or an induction heater (30).

13. The device according to claim 12, **characterized in that** a heating wire (16) is wound around a core (2), which core (2) surrounds a fluid path of the fluid within the hand piece (112).

14. The device according to claim 13, **characterized in that** the core (2) has a bore (8) for guiding the fluid through the core (2) and an outer circumference provided with a helical groove (14), wherein a resistance heating wire (16) wound around the core (2) and received with the groove (14).

15. The device according to claim 14, **characterized in that** the heating wire (16) and/or the core (2) is coated with an electrically insulating material.

16. The device according to claim 14 or 15, **characterized in that** the core (2) is made of an electrically insulating material.

17. The device according to one of the preceding claims, **characterized in that** the hand piece (112) comprises a nozzle component (4) provided with a nozzle element (18), a connector component (6) provided adapted to connect to a tube (110), fluidically connecting the hand piece (112) with the pump (100), and a core (2) surrounded by the heating means (16, 30), which core is interdisposed between the nozzle component (4) and the connector component (6).
